# EUROPEAN PATENT APPLICATION

(11) **EP 3 269 359 A1**
(43) Date of publication of application: **17.01.2018**
(21) Application number: 17425074.6
(22) Date of filing: 11.07.2017
(51) Int. Cl.: A61K 9/00, A61K 9/14, A61K 33/38, A61K 47/06, A61K 47/10, A61K 47/36, A61K 47/38

(54) **USE OF SILVER NANOPARTICELS ANTIBATTERY ACTIVITY IN HUMAN AND ANIMAL USE FORMULATIONS**

(30) Priority: 12.07.2016 IT 201600072510
(71) Applicant: Omini, Gabriele, 20064 Gorgonzola (MI) (IT)
(72) Inventor: Omini, Gabriele, 20064 Gorgonzola (MI) (IT); Omini, Caludio Franco, 20060 Bussero (MI) (IT)

(57) **Abstract**

The use of silver nanoparticles (AgNP) with diameter <30 nm and negative zeta potential with powerful antibacterial and antifungal activities suitable for pharmaceutical preparations administrable to humans and/or animals on the need. In particular formulations containing AgNP with the following features are claimed and particularly preferred: an effective diameter of 18.6 ± 2.3 nm evaluated by transmission electron microscopy (TEM); a mean hydrodynamic diameter of 42.3 ± 1.2 nm evaluated by dynamic light scattering and of 40.5 ± 0.6 nm evaluated by nanoparticle tracking analyzer (NanoSight); a centrifugal density size of 17.1 ± 0.7 nm evaluated by differential centrifugal sedimentation; a Z potential of -20.9 ± 3.7 mV as measured by DLS-Zeta Potential. Such nanoparticles are largely more potent, at the same concentration of Ag ions, and with greater duration of antibacterial/antifungal activity as compared to standard AgNP currently available. Furthermore, the AgNP claimed retain their antibacterial/antifungal efficacy both in aqueous and non-aqueous formulations, after freeze-drying processes; they are of easy synthesis and have a scars and have a low toxicity for eukaryotic cells.

## Description

**Field of Application:** The term nanoparticles refers to systems of sizes between 1 and 100 nm, but recently this concept has been extended to systems up to micron size. Compounds of sizes between 1 and 100 nm have chemical-physical, magnetic, optical properties that are extraordinarily unique in this dimensional field. Colloidal silver nanoparticles have been used in the medical field to prevent and treat various diseases, especially infections.

**OBJECT OF THE INVENTION:** The object of the present invention is the identification, production and use of silver nanoparticles suitable for the preparation of pharmaceutical preparations for human and / or animal administration for the prevention and treatment of bacterial / mycotic infections of various origin. Another object of the present invention is the use of silver nanoparticles as preservatives and preservatives for the microbiological integrity of pharmaceutical formulations.

**State of the art:** Discovery of antibiotic drugs has been one of the major victories in science, contributing considerably to the prolonged life expectancy of the world's population. However with the increasing use and abuse of antibiotic drugs has produced the phenomenon of cross-resistance. More and more pathogenic bacteria have become resistant to the action of antibiotic drugs. This effect is currently a major medical problem so much that worldwide research is engaged in identifying antibacterial molecules that can overcome cross-resistance. In this regard, the use of non-traditional antibacterial compounds is of particular relevance *(*Franci G. et al., Silver Nanoparticles as Potential Antibacterial Agents. Molecules 2015, 20, 8856-74*).* Colloidal silver nanoparticles currently have large and diverse applications in various fields, including optics and photonics, electronic microscopy, catalysis and biochemistry, and are found in everyday products such as computers, cell phones, phones and vacuum cleaners, filters for Water and air, textile products and, thanks to antibacterial and antimicrobial properties, also in deodorants, soaps, toothbrushes, toothpastes. Silver has been used for centuries against various pathologies, and in particular for its outstanding antiseptic and antimicrobial efficacy, both against Gram + and Gram-associated bacteria associated with a low overall toxicity. Although the mechanism of action is not yet fully known and partly controversial, it is now well known that silver ions have very powerful antimicrobial properties linked to the blockage of the enzymatic respiratory system, alteration of microbial DNA and degradation of the cellular wall. At the same time, art is well-known in the antibacterial efficacy of silver nanoparticles having a broad spectrum of action against morphologically and metabolically different microorganisms. These differences associated with a common efficacy of silver nanoparticles allow to hypothesize an interaction with unique and underestimated bacterial membranes to exploit. In particular, the chemical-physical properties of silver nanoparticles allow their interaction with the surface of the bacterial cell by modifying its structure and favoring the permeability and subsequent death of the bacterial cell *(*Franci G. et al., Silver Nanoparticles as Potential Antibacterial Agents. , 20, 8856-74*).*

**Definitions:** Below are the definitions of the main terms used in the present invention: 1) silver nanoparticles (here below as also referred as AgNP) are particles composed by clusters of silver atoms of 1-100 nm in size; 2) pharmaceutical formulations are intended to be as known in the art for the administration of any active principle useful for the treatment, prevention of a disease and maintenance of the physiological and anatomical activities in humans and animals. Also included are formulations that can be used for cosmetic or food purposes; 3) preservative means the excipient introduced in the pharmaceutical form to prevent pollutants from compromising the microbiological stability of the preparation; 4) hydrodynamic diameter is the nanoparticles size and is related also to the aggregation state; 5) Zeta potential is the measurement of the repulsive force between the particles and it is an index of their colloidal stability; 6) *dynamic light scattering* (DLS) and *nanoparticle tracking analyzer (NanoSight)* are two well-known laboratory instruments (Vasco F et al. Critical evaluation of nanoparticle tracking analysis (NTA) by NanoSight for the measurement of nanoparticles and protein aggregates. Pharmaceutical Res. 2010, 27 (5), 796-810*),* that measure the nanoparticles average hydrodynamic diameter which is the size of the nanoparticle in the solvent in which it is dispersed and it is also indicative of its stability and aggregation. *Differential centrifugal sedimentation (DCS)* measures the diameter of a particle relative to its centrifugal density.

**Description:** In order to obtain AgNP suitable for the manufacture of pharmaceutical formulations for mammalian administration, including man, methods of physical preparation of the same have been selected. Example 1 shows the synthesis and characterization of nanoparticles that gave the most interesting results. Through the methodology described in the aforementioned example (example 1), though already known in art, a surprising and unexpected result was obtained. In fact, as shown in Example 2, the AgNPs claimed in the present invention exhibit an antibacterial efficacy significantly greater than that shown in the same experimental conditions as commercially available nanoparticles. This unforeseeable result from what is known in the art is quite astonishing since silver ion concentrations are equivalent to all of the nanoparticles compared, and the size of the same is also comparable. Indeed, it is obvious that the antibacterial activity of said nanoparticles is related to the action of silver ions and therefore at the same concentration of silver ions, the effectiveness should be similar. Also the size of the nanoparticles is similar in fact is known in the art as the optimal size of AgNPs to exert antibacterial efficacy should be <30 nm and all the nanoparticles tested are in this order of magnitude. *(*Franci G. et al., Silver Nanoparticles as Potential Antibacterial Agents, Molecules 2015, 20, 8856-74*).* Another novelty of the present invention relates to the peculiar feature of the selected nanoparticles, as is known in the art as an essential condition for facilitating the efficacy of AgNPs to exert antibacterial activity and is also linked to the positivity of zeta potential. Positive charge allows a more effective electrostatic interaction with negative bacterial wall charges *(see page 8858 last paragraph in* Franci G. et al., Silver Nanoparticles as Potential Antibacterial Agents, Molecules 2015, 20, 8856-74*).* As widely demonstrated by our studies and reported in Example 1, the selected AgNPs have a negative potential (-20.9 ± 3.7 mV) that theoretically should be, according to what is known in the art, deleterious to the efficacy of the invention. This obviously contrasts with the experimental data we found and gives rise to further novelties as we have claimed. In fact, in the present invention AgNP is claimed having an average diameter of less than 22 nm and more preferred AgNP with a diameter between 15 and 21 nm. Such AgNPs may have a potential negative zeta and particularly preferred AgNPs with zeta potential less than -25 mV and even more particularly preferred AgNPs with zeta potential values between -17 mV and -24 mV. This novelty element is another important benefit in terms of potential toxicity to mammalian cells including human and in vitro and in vivo. It is widely documented that cationic nanoparticles exhibit high cytotoxicity, while neutrally-neutralized or negatively charged negative charges *(*E. Fröhlich, The role of surface charge in cellular uptake and cytotoxicity of medical nanoparticles. Int. J. Nanomed 2012, 7, 5577-91*).*

Indispensable feature for all pharmaceutical formulations is their stability, that is, over time, the formulation as a whole and in particular the active ingredients do not undergo chemical-physical degeneration. The AgNPs we claim have excellent chemical-physical stability qualities. As shown in Example 1, the AgNPs claimed by us have a stability in aqueous solution of at least one year. Moreover, the possibility of lyophilization of the claimed AgNPs allows them to be stored, in optimal conditions, for very long and potentially indefinable periods of time. The preparation of local topical pharmaceutical formulations suitable for administration to humans and animals may require the use of liposoluble or water soluble principles based on the formulation requirements. AgNPs as in Example 1 are obtained in aqueous environment and are therefore readily usable in water-soluble pharmaceutical formulations. On the other hand, the selected and claimed AgNPs can be suitably and conveniently transferred to a lipophilic environment as described in Example 3. Therefore, the selected and claimed AgNPs are suitable for the manufacture of solid pharmaceutical formulations (powders, granules, tablets, Capsules, etc.), liquid and semisolid (aqueous and oily solutions, gels, creams, lotions, etc.) without loss of antibacterial efficacy as described in Example 2. The preparation of pharmaceutical formulations for human or animal use cannot disregard to checking that the risk of toxicity is acceptable for human or animal use. The selected and claimed AgNPs of the present invention exhibit a totally acceptable and comparable toxicity to that expressed by silver ions regularly used in cosmetic, pharmaceutical and food products freely marketed. Example 4 demonstrates the virtually absence of toxic effects of the selected and claimed AgNPs on in vitro murine fibroblasts evaluated by a classic reference test for cytotoxic activity (MTT). Therefore, in the present invention are claimed AgNP prepared by known on the art chemical or physical procedures, or by innovative methodologies, particularly preferred for their production are chemical syntheses that produce AgNP characterized by: uniform dimensions below 30 nm, defined composition above 99 %, high reproducibility of synthesis and properties claimed, prolonged colloidal stability (over 12 months). The nanoparticles claimed in the present invention are suitable for the preparation of pharmaceutical formulations (medicines, medical devices), cosmetics and nutraceuticals (food supplements) and foodstuffs as preservatives for the prevention of formulation from the development of microorganisms (bacteria, fungi). Such formulations may be of various type and are well-known in the art including liquid or non-aqueous liquid formulations (organic and lipid solvents) provided they are suitable for administration to humans and animals in accordance with current regulations. By way of example only liquid or semisolid compositions containing AgNP mainly water-based, as aqueous solutions, gels, oil/water emulsions are claimed. In the present invention, compositions containing AgNP are also claimed, wherein the usable excipients are lipophilic, insoluble or semi-soluble in water, miscible or semi-immiscible in oil and other organic solvents (vaseline, glycerol, polymers, etc.) provided they are suitable for the production of oily solutions and suspensions, oil/water emulsions, creams and ointments and to be administered to man or animal. Such formulations can also be used topically, topically on mucous, cutaneous, orally, parenterally, and applied to the natural orifices of the body in subjects with infectious bacterial pathologies, whether supported by Gram + and/or Gram- bacteria, fungi, for their care, therapy and prevention. Additionally, if desired, such formulations may suitably be applied and/or adhered to rigid or flexible surfaces that come into contact with the human or animal body, or inserted into natural orifices, implanted, semi- or permanently- implanted in organs and tissues of human or animal.

### EXAMPLE 1 - AgNP SYNTHESIS AND CHARACTERIZATION

### Synthesis in aqueous solution

Silver colloidal nanoparticles (AgNP) are synthesized in aqueous solution following the published protocol of N. G. Bastús, F. Merkoçi, J. Piella, V. Puntes Synthesis of highly monodisperse citrate stabilized silver nanoparticles of up to 200 nm: kinetic control and catalytic properties. Chem. Mater., 2014, 26 (9), 2836-2846.

Briefly, an aqueous solution (100 mL) containing sodium citrate (SC 5 mM) and tannic acid (0.025 mM) was prepared and heated to the boiling point (an evaporator condenser is used to avoid evaporation of the solvent). The solution was boiled for 15 min, and then 1 mL of aqueous silver nitrate solution (25 mM) was quickly added with a syringe under vigorous stirring (750 rpm). The solution became immediately yellow, and after 1 h heating, it was allowed to cool down and kept under stirring overnight at room temperature.

AgNPs formation was assessed using ultraviolet-visible (UV-vis) spectroscopy by the occurrence of the characteristic absorption peak (around 400 nm) corresponding to the plasmon resonance band. The resulting AgNPs were purified by centrifugation to eliminate the unreacted materials and further suspended in adequate water solvent, depending on the use.

### NPs characterization

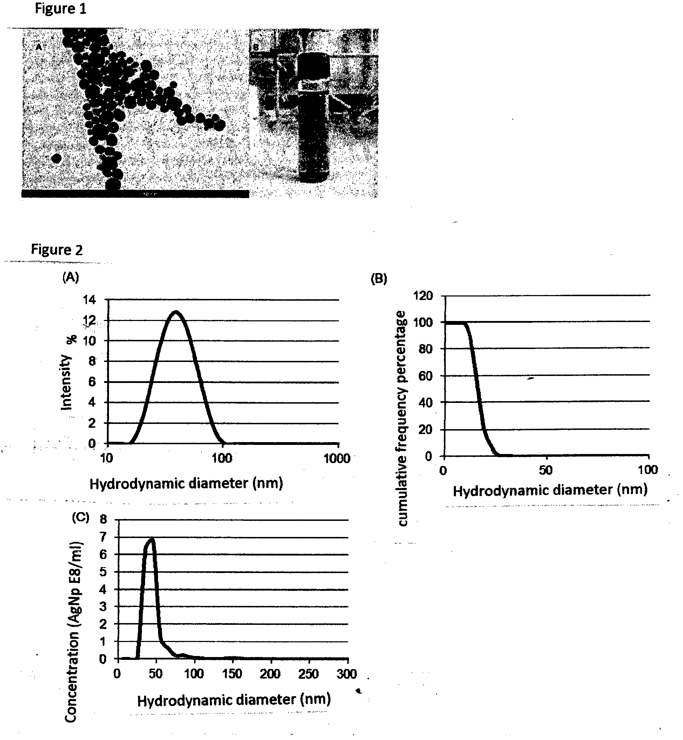

The AgNPs were determined through transmission electron microscopy (TEM). (Figure 1 Image obtained by Transmission Microscope (TEM) of AgNP suspended in water and deposited on grid (A); AgNP in solution (B)); The AgNPs showed a mean effective diameter of 18.6±2.3 nm (PDI 0.143 ± 0.053) and a mean hydrodynamic size of 42.3 ± 1.2 nm (PDI 0.231 ± 0.034) evaluated by dynamic light scattering DLS, 40.5 ± 0.6 nm evaluated by nanoparticle tracking analyzer (Nanosight) and a centrifugal density size of 17.1 ± 0.7 nm obtained by differential centrifugal sedimentation (DCS) (Figure 2 Dimensional characterization of AgNP by (A) dynamic light scattering (DLS) in 1 mM citrate buffer; (B) nanoparticle tracking analyzer (Nanosight) in sterile water; (C) differential centrifugal sedimentation (DCS) in sucrose gradient). AgNPs were negatively charged with a zeta potential of-20.9 ± 3.7 mV calculated by electrophoretic mobility by DLS-Zeta Potential.

### Stability in solution

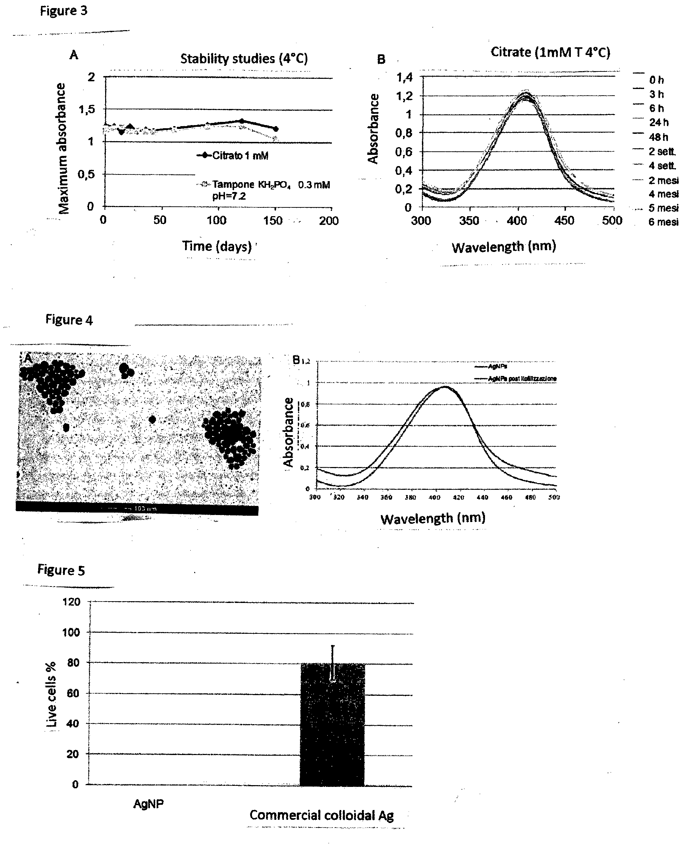

The long-term stability of AgNPs was assessed by monitoring the NPs plasmon band position of AgNPs by UV-vis absorption spectroscopy. Either 1 mM citrate buffer solution or 0.3 mM phosphate buffer solution, pH 7.2 was used The AgNP were stable in water solution (Figure 3 Long term stability studies of AgNP in 1 mM citrate buffer and 0.3 mM phosphate buffer pH 7.2 (A); Absorption spectrum of AgNP silver plasmon (B).) In further studies no degradation or aggregation was observed over 12 months of storage and after thermal stress (60 °C for 6 months), which may confirm a conventional stability over 36 months.

The integrity and colloidal stability of AgNPs was confirmed by evaluating the size of the NPs dispersion after lyophilization and resuspension (Figure 4 TEM image of AgNP lyophilized and suspended in citrate buffer (A) and absorption spectrum of AgNP before and after lyophilization (B)). Prior to lyophilization, post-synthesis samples are purified as described above and suspended in a 1 mM aqueous citric acid solution.

### EXAMPLE 2 - ANTIBACTERIAL ACTIVITY OF AgNPs

### Antibacterial activity of AgNPs against in E. coli MG1665 and comparison with commercialy available systems

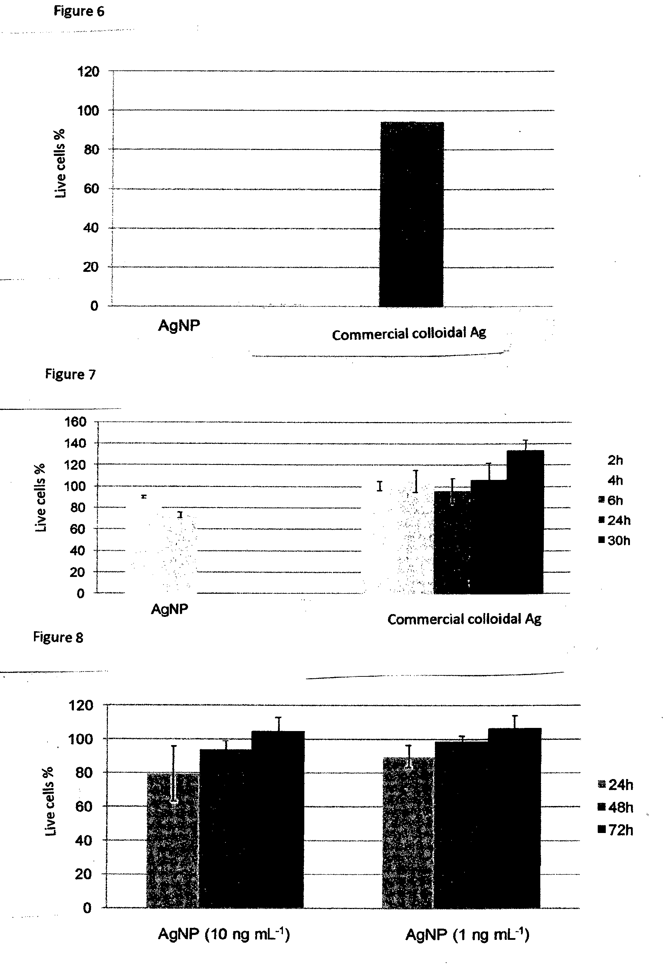

Bacterial cultures were grown overnight in nutrient broth, then the suspension was diluted at an initial optical density of 0.1 at 600 nm (OD600) and cultures were allowed to grow at 37°C under stirring. When the cultures reached an OD600 = 0.65 was withdrawn, centrifuged, and washed twice with 0.3 mM PB, pH 7.2. Following washing, the cells were suspended and diluted in phosphate buffer containing AgNPs or other antibacterial agents to get a final bacterial concentration of 2×105 CFU/mL and were incubated at 37°C for 2 h. After the incubation time, samples up to 2×102 theoretical cells number were plated on a solid growth medium and incubated overnight at 37°C. The number of CFU grown on the plate was counted and the results expressed as difference in viability in comparison to the untreated control (Figura 5 Antibacterial activity tests performed by incubating AgNP (0.54 ng mL-1 content of Ag ions) with E. coli MG1665 for 2 h.). The AgNPs claimed showed a superior antibacterial efficacy compared with the above-mentioned commercial AgNPs (Santè Colloidal Silver - Sante Naturels, Argento colloidale - OligoNutriva, Argento Colloidale puro - Hydromed, Silver nanoparticles - Sigma Aldrich). Same results were obtained with Gram + bacteria (S. Aureus, Figure 6 Antibacterial activity tests performed by incubating AgNP (5.4 ng mL-1 content of Ag ions) with S. Aureus for 2 h) and fungi (Candida Albicans).

### Long-term antibacterial activity

AgNPs with a low antibacterial concentration were incubated with 2×105 E. coli cells for 2, 4, 6, and 24 h. After the incubation time, 2×102 theoretical cell numbers of each sample were plated on a solid growth medium and incubated for 12 h at 37°C.

AgNPs claimed exhibited a significantly higher efficacy as compared to other tested Ag product (Figure 7 Antibacterial activity tests performed by incubating AgNP for 2, 4, 6, 24, 30 hours (0.54 ng mL-1 Ag ions content) with E. coli MG1665.).).

### AgNPs phase transfer in oil phase and antimicrobial properties maintenance

Tests analog to those described above were carried out using AgNPs soluble in oil phase. THF was chosen as an organic solvent to suspend AgNPs for its miscibility with a cell medium. The obtained data showed that the antibacterial efficacy of AgNPs was retained even when AgNPs were rendered suitable for the dispersion in oil. Same results were obtained using in vitro preparations of Gram- and Gram+ bacteria or fungi

### EXAMPLE 3 - AgNP OIL PHASE TRANSFER

The AgNPs may be transferred by water phase into the organic phase according to the following procedure: 10 mL Octadecylamine solution 5.4 mg/mL (0.02 M) in ethanol was added while stirring to 10 mL of AgNPs solution (0.1 nM) obtained as described above. After 3-5 min, toluene (10 mL) was added to the mixture and left under stirring for 10 minutes. The solution is dried an washed twice to remove the excess alkylamine by precipitating and resuspending nanoparticles in ethanol. Thereafter the solvent may be evaporated and resuspended in the selected solvent depending on the use.

### EXAMPLE 4 - AgNP TOXICITY TESTs

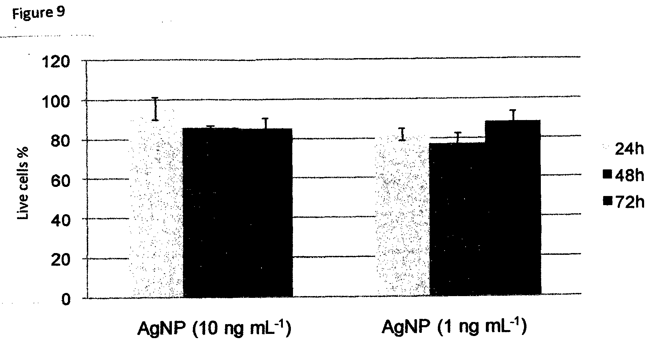

An MTT assay was performed to evaluate the toxicity of AgNPs. Water- or THF-suspended AgNPs were incubated at effective antimicrobial concentrations with 3T3-L1 murine fibroblasts. AgNPs in water solution or ready to oil suspension and suspended in THF did not display significant cytotoxicity at the concentrations tested (Figures 8 MTT test was performed by incubating AgNP (10 and 1 ng mL-1 content of Ag ions) suspended in water with 3T3-L1 murine fibroblasts. Figure 9 MTT test performed by AgNP incubation (10 and 1 ng mL-1 content of Ag ions) suspended in THF with 3T3-L1 murine fibroblasts).

### EXAMPLE 5 AgNP Pharmaceutical Formulations

### Water-soluble formulations for antibacterial activity

A) AgNP 0.001%; Hydroxyethylcellulose 3.0%; H2O q.b. 100 g
B) AgNP 0.003%; Hyaluronic Acid (Sodium Salt) 0.3%; Hydroxyethylcellulose 2.0%; H2O q.b. 100 g
C) AgNP 0.002%; Carboxymethylcellulose 0.3%; Glycerol 2%; Sorbitol 4%; Glycerin 2%; H2O q.b.
100 g

### Liposoluble antibacterial formulations

A) AgNP 0.002%; Cetylstearyl alcohol 5%; 5% stearic acid; PEG 3%; Vaseline oil 20%; White iron 40%; H2O q.b. 100 g
B) AgNP 0.0005%; EPA / DHA 5%; Hyaluronic Acid (Sodium Salt) 0.2%; Stearyl alcohol 5%; 20% stearic acid; Isopropyl melted 10%; H2O q.b. 100 g
C) AgNP 0.001%; EPA / DHA 10%; Vaseline oil 20%; PEG q.b. 100g

## Claims

1. Use of silver nanoparticles having antibacterial/antimycotic activity **characterized by**: the dimension of nanoparticles less than 30 nm; and with negative zeta potential suitable for human or animal administration

2. Use of silver nanoparticles according to claim 1 further **characterized by**:
a mean effective hydrodynamic diameter of 18.6 ± 2.3 nm (PDI 0.143 ± 0.053 e zeta potential of -20.9 ± 3.7 mV

3. Use of silver nanoparticles according to the preceding claims soluble in aqueous solvents and/or soluble or dispersible in non-aqueous solvents suitable for preparing formulations for human and/or animal use

4. Use of silver nanoparticles according to the preceding claims obtained after lyophilization.

5. Use of silver nanoparticles according to the preceding claims in liquid, semisolid and solid pharmaceutical formulations with antibacterial and antifungal activities based on water-soluble, fat-soluble ingredients and their combinations.

6. Use of silver nanoparticles according to the preceding claims in food, cosmetic, nutraceutical and pharmaceutical formulations for the conservation and preservation of their microbiological integrity even in combination with other preservatives.

7. Use of silver nanoparticles according to the preceding claims having antibacterial activity, including Gram+ and Gram- bacteria, antifungal activity including fungi and yeasts present in the subject to which are administered

8. Use of silver nanoparticles according to the preceding claims in liquid, semisolid and solid formulations suitable to be administered by topical route including on intact or injured skin and mucosae, oral route, parenteral route, and on the natural orifices of the body to a human or animal subject on the need.

9. Use of silver nanoparticles according to the preceding claims having antibacterial and antifungal activity applied on rigid or flexible surfaces that are in contact with the with the human or animal body, that are plug in natural orifices or implanted semi- or permanently or in human or animal organs and tissues.
